(19) Europäisches Patentamt
European Patent Office
Office européen des brevets

(11) Veröffentlichungsnummer: **0 127 835**
**B1**

(12) # EUROPÄISCHE PATENTSCHRIFT

(45) Veröffentlichungstag der Patentschrift: **13.08.86**

(51) Int. Cl.⁴: **C 07 C 45/74**, C 07 C 45/73, C 07 C 49/04

(21) Anmeldenummer: **84105829.0**

(22) Anmeldetag: **22.05.84**

(54) **Verfahren zur Herstellung von Ketonen.**

(30) Priorität: **28.05.83 DE 3319430**

(43) Veröffentlichungstag der Anmeldung: **12.12.84 Patentblatt 84/50**

(45) Bekanntmachung des Hinweises auf die Patenterteilung: **13.08.86 Patentblatt 86/33**

(84) Benannte Vertragsstaaten: **BE CH DE FR GB IT LI**

(56) Entgegenhaltungen:
**EP-A-0 028 703**
**CH-A-629 170**
**DE-A-2 023 512**

**HOUBEN-WEYL "Methoden der organischen Chemie", 4. Auflage, Band VII/1, 1954 GEORG THIEME VERLAG, Stuttgart Seite 389**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Gramlich, Walter, Dr., Auf der Hoehe 11, D-6803 Edingen- Neckarhausen (DE)**
Erfinder: **Hoffmann, Werner, Dr., Ringstrasse 11c, D-6708 Neuhofen (DE)**
Erfinder: **Hupfer, Leopold, Dr., Waltershoehe 3, D-6701 Friedelsheim (DE)**
Erfinder: **Merger, Franz, Dr., Max- Slevogt- Strasse 25, D-6710 Frankenthal (DE)**
Erfinder: **Paetsch, Juergen, Dr., Am Altenbach 30, D-6706 Wachenheim (DE)**

**EP 0 127 835 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von Ketonen durch Umsetzung eines ungesättigten Aldehyds mit einem Keton und Wasserstoff an einem Katalysatorsystem.

Bekanntlich lassen sich ungesättigte Aldehyde in Gegenwart heterogener Katalysatoren unter Absättigung einer CC-Doppelbindung zu gesättigten Aldehyden hydrieren. Ferner ist bekannt, daß man die Dimerisierung von Ketonen, wie Aceton oder die Dimerisierung von Aldehyden unter hydrierenden Bedingungen in einem Verfahrensschritt so durchführen kann, daß eine gesättigte höhere Carbonylverbindung erhalten wird. Bei diesen, z.B. in den deutschen Offenlegungsschriften 2 023 512 und 2 022 365, den deutschen Auslegeschriften 1 238 453 und 1 936 203 und der US—PS 3 379 766 beschriebenen Verfahren werden wegen der dadurch bedingten einfachen Reaktionsführung Festbettkattlysatoren bevorzugt. Es werden sowohl Reaktionen in der Gasphase als auch in der Flüssigphase beschrieben, wobei eine Umsetzung in der flüssigen Phase wegen der hierbei erreichbaren längeren Kontaktstandzeiten zu bevorzugen ist, insbesondere dann, wenn teure Katalysatoren, wie solche mit Edelmetallkomponenten, verwendet werden.

In der DE—OS 2 615 308 wird die Umsetzung von gesättigten aliphatischen oder aromatischen Aldehyden mit Methylketonen in Gegenwart eines Katalysatorsystems beschrieben, das einerseits ein Oxid der seltenen Erdmetalle und andererseits ein oder mehrere Metalle der Gruppe VIII des Periodensystems der Elemente enthält. Die nach diesem Verfahren erhältlichen Ketone sind, insbesondere dann, wenn man von verzweigten aliphatischen Aldehyden ausgeht, gesuchte Lösungsmittel für Lacke sowie begehrte Riechstoffe oder Riechstoffvorprodukte. Von Nachteil ist es, daß die dazu benötigten verzweigten aliphatischen Aldehyde oft sehr schwer zugänglich sind. In den meisten Fällen werden sie durch Hydroformylierung der entsprechend substituierten Alkene hergestellt. Die Hydroformylierung laßt sich aber nicht völlig regioselektiv durchführen, und die entstandenen diastereomeren Aldehyde sind meist nur sehr schwierig zu reinigen. Erschwerend kommt noch hinzu, daß das in die Hydroformylierung einzusetzende Alken häufig ebenso stereochemisch uneinheitlich ist und als Diastereomerengemisch vorliegt.

Um stereochemisch einheitliche, verzweigte Aldehyde zu erhalten, empfiehlt es sich deshalb häufig, diese über die Stufe der α,β-ungesättigten Aldehyde durch katalytische Hydrierung zu synthetisieren. Stereochemisch einheitliche α,β-ungesättigte Aldehyde lassen sich z. B. aus gesättigten Aldehyden durch Aldolkondensation mit Formaldehyd nach bekannten Verfahren (Houben-Weyl, Methoden der organischen Chemie, Band 7/1, Seiten 89 bis 92) herstellen.

Es wurde nun gefunden, daß man Ketone der Formel

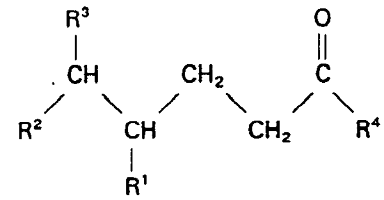

I

in der $R^1$, $R^2$ und $R^3$ Wasserstoffatome, Alkylgruppen mit 1 bis 10 C-Atomen, die durch Alkoxy- oder Arylgruppen substituiert sein können, Arylgruppen, Cycloalkylgruppen oder heterocyclische Reste mit 6 bis 20 C-Atomen, die durch Alkylgruppen substituiert sein können, bedeuten, $R^4$ die gleiche Bedeutung hat, jedoch nicht für ein Wasserstoffatom steht und $R^1$ und $R^2$ auch Reste sein können, die mit den C-Atomen an die sie gebunden sind, einen cycloaliphatischen oder heterocyclischen Ring bilden, vorteilhaft dadurch herstellen kann, daß man Aldehyde der Formel

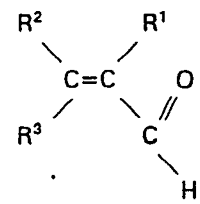

II

in der $R^1$, $R^2$ und $R^3$ die obengenannte Bedeutung haben, mit Ketonen der Formel

$$H_3C{-}CO{-}R^4 \qquad III$$

in der $R^4$ die obengenannte Bedeutung hat, bei höherer Temperatur und in einer Stufe in Gegenwart von Wasserstoff und eines Katalysatorsystems, das einerseits ein Oxid oder Phosphat der seltenen Erdmetalle, des Magnesiums, Aluminiums, Zinks oder Titans, und andererseits ein Metall der Gruppe VIII des Periodensystems der Elemente enthält, umsetzt.

Nach dem erfindungsgemäßen Verfahren erhält man aus den α,β-ungesättigten Aldehyden und den Ketonen überraschenderweise die gewünschten höheren Ketone, ohne daß in merklichem Ausmaß Nebenreaktionen eintreten. Aufgrund der großen Reaktionsfähigkeit der α,β-ungesättigten Aldehyde (Ullmann, Encyclopädie der technischen Chemie 4. Auflage, Band 7 [1974] Seiten 74 und 132) war zu erwarten, daß unter den Reaktionsbedingungen Nebenreaktionen auftreten würden, wie

1. Polymerisationsreaktionen (Ullmann, Encyclopädie der technischen Chemie 4. Auflage, Band 7, Seite 74),
2. [4+2] - Cycloadditionsreaktionen (Houben-Weyl, Methoden der organischen Chemie, Band VII/1, Seite 130) zu substituierten Dihydropyranen,

3. Michael-Additionen des CH-aciden Methylketons an die α,β-ungesättigte Doppelbindung (Houben-Weyl, Methoden der organischen Chemie, Band VII/1, Seite 96),
4. Homoaldolreaktionen zwischen zwei Molekülen der Methylketonkomponente (DE—AS 1 238 453),
5. Decarbonylierungsreaktionen der eingesetzten Aldehyde (vgl. J. Org. Chemistry *24*, Seite 1369 [1959].

Außerdem mußte es überraschen, daß trotz der für die einstufige Umsetzung erforderlichen hohen Wasserstoffzudosierung die beiden Partialhydrierungen, insbesondere die des α,β-ungesättigten Aldehyds, selektiv und ohne nennenswerte Weiterhydrierung unter Bildung von Alkoholen ablaufen. Durch Maßnahmen, wie sie bei der Hydrierung α,β-ungesättigter Aldehyde normalerweise üblich sind, wie das Arbeiten bei niedrigen Temperaturen (vgl. Houben-Weyl, Methoden der organischen Chemie, Band VII/1, Seite 389) und die Empfehlung, die Partialhydrierung nach Aufnahme von einem Mol Wasserstoff abzubrechen, war das Verfahren der Erfindung nicht zu verwirklichen.

In den Aldehyden der Formel II und den Ketonen der Formel III bedeuten die Reste $R^1$, $R^2$ und $R^3$ vorzugsweise Wasserstoffatome, verzweigte oder unverzweigte Alkylgruppen mit 1 bis 10, insbesondere 1 bis 5 C-Atomen oder Arylgruppen. $R^4$ kann für eine der genannten Gruppen stehen, kann aber kein Wasserstoffatom bedeuten. $R^1$ und $R^2$ können außerdem auch Reste sein, die mit den C-Atomen, an die sie gebunden sind, einen cycloaliphatischen oder heterocyclischen Ring mit 5 bis 8 C-Atomen, wie einen Cyclohexen- oder einen Dihydropyranring bilden.

Als Aldehyde der Formel II seien beispielsweise genannt:

Acrolein (Propenal), Methacrolein, 2-Ethylpropenal, Crotonaldehyd, 2-Pentenal, 2 - Ethyl - 2 - hexenal, Zimtaldehyd, Cyclohexencarbaldehyd und 5,6 - Dihydropyran - 2 - carbaldehyd. Als Ketone der Formel III lassen sich beispielsweise Aceton, 2-Butanon, Acetophenon und Methyl-cyclohexylketon verwenden.

Nach dem neuen Verfahren werden die Aldehyde mit den Ketonen in einer Stufe, z.B. bei Temperaturen zwischen 100 und 280°C, vorzugsweise bei 150 bis 230°C, in Gegenwart von Wasserstoff und dem Katalysatorsystem umgesetzt. Zweckmäßig arbeitet man bei Drücken von 10 bis 60 bar, vorzugsweise 20 bis 40 bar. Gegebenenfalls arbeitet man in Gegenwart eines Stabilisators. Als Stabilisatoren für die α,β-ungesättigten Aldehyde haben sich z.B. Hydrochinon und Hydrochinonmonomethylether bewährt, wobei ppm-Mengen ausreichen.

Das erfindungsgemäß verwendete Katalysatorsystem enthält einerseits ein Oxid oder Phosphat der seltenen Erdmetalle, des Magnesiums, Aluminiums, Zinks oder Titans und andererseits ein oder mehrere Metalle der Gruppe VIII des Periodensystems der Elemente. Als Oxide der seltenen Erdmetalle, die man auch im Gemisch anwenden kann, werden bevorzugt Lanthanoxid ($La_4O_6$), Ceroxid ($CeO_2$), Praseodymoxid ($Pr_4O_6$) und Neodymoxid ($Nd_4O_6$) verwendet. Von den Metallen der Gruppe VIII des Periodensystems werden bevorzugt Palladium und Platin verwendet.

Man kann die Katalysatoren in reiner Form oder auf einem inerten Träger verwenden. Als inerte Träger lassen sich beispielsweise Aluminiumoxid, Aluminiumsilikat, Aktivkohle, Magnesiumsilikat und Siliciumdioxid, wie Kieselgel oder Kieselgur verwenden. Vorzugsweise werden Träger auf Basis Aluminiumoxid oder Böhmit verwendet. Der Gehalt an den Oxiden oder Phosphaten der seltenen Erdmetalle oder der Metalle Mg, Al, Zn oder Ti, bezogen auf das Trägermaterial, kann von 0,5 bis 20 Gew.% variieren. Der Gehalt an den Metallen der Gruppe VIII des Periodensystems, bezogen auf das Trägermaterial, kann bei Palladium und Platin z.B. zwischen 0,05 und 5 Gew.% schwanken. Bei Verwendung von Ni oder Co ist ein Gehalt von z.B. 1 bis 30 Gew.% zweckmäßig.

Das Aufbringen der aktiven Kompenten auf den Träger kann entweder getrennt oder aber gemeinsam nach Vermengung erfolgen. Desweiteren kann es durch Aufbringen erst im Reaktor erfolgen.

Die nach dem Verfahren der Erfindung erhältlichen Ketone sind interessante Lösungsmittel sowie Riechstoffe und Pharma-Vorprodukte.

Beispiel 1

Als Apparatur dient ein mit Kontakttabletten gefüllter 2,5 1-Rohrreaktor:

Die Kontakttabletten haben einen Durchmesser von 4 mm und besitzen folgende Zusammensetzung: 0,5 Gew.% Palladium, 5 Gew.% Praseodymoxid auf -Aluminiumoxid. Über der Kontakt leitet man pro Liter Kontakt und pro Stunde bei einem Druck von 25 bar ($H_2$-Atmosphäre) und 180°C 1 l eines Gemisches aus 73 Gew.% Aceton und 27 Gew.% 2-Ethylpropenal, das 2 ppm Hydrochinon enthält. Der Reaktionsaustrag enthält:

| | |
|---|---|
| Aceton | 44 Gew.% |
| 2-Ethylpropenal | 0,2 Gew.% |
| 2-Methylbutanal | 2,2 Gew.% |
| Isopropanol | 0,2 Gew.% |
| 2-Methylbutanol | 0,1 Gew.% |
| 4-Methyl-2-pentanon | 1,8 Gew.% |
| 5-Methyl-2-heptanon | 45 Gew.% |
| 5-Methyl-3-hepten-2-on | 0,8 Gew.% |
| 3,9-Dimethyl-6-undecanon | 3,1 Gew.% |

Beispiel 2

In einem Hydrierautoklaven werden 470 g 3 - Methyl - 2 - butanon, 420 g 3 - Methyl - 2 - butenal und 50 g eines heterogenen Katalysators, der 1 Gew.% Palladium auf Magnesiumoxid enthält, unter Stickstoff vorgelegt. Nach Spülen mit Wasserstoff wird bei Raumtemperatur 20 bar $H_2$-Druck aufgepreßt. Man hydriert 3 Stunden bei 120°C und 30 bar Wasserstoffdruck, 3 Stunden bei 160°C und 40 bar Wasserstoffdruck und 6 Stunden bei 200°C und 50 bar Wasserstoffdruck. Nach Abtrennen des Katalysators erhält man 625 g 2,7 - Dimethyl - 3 - octanon, das entspricht einer Selektivität (bezogen auf das Methylbutenal) von 80% der Theorie.

Beispiel 3

In einem Autoklaven werden unter Stickstoff 87 g Aceton (1,5 Mol), 63 g 2 - Ethyl - 2 - hexenal (0,5 Mol) sowie 8 g eines Heterogenkatalysators, der 0,7 Gew.% Palladium und 3 Gew.% $CeO_2$ auf Aluminiumoxid enthält, vorgelegt. Dann wird mit Wasserstoff gespült und ein Wasserstoffdruck von 20 bar aufgepreßt. Man hydriert jeweils 3 Stunden bei 90°C/25 bar Wasserstoffdruck, 150°C und 50 bar sowie 190°C und 55 bar Wasserstoffdruck. Nach Abtrenen des Katalysators erhält man durch Aufdestillation 64 g 5 - Ethyl - 2 - nonanon, das entspricht einer Selektivität (bezogen auf Ethylhexenal) von 75%.

**Patentansprüche**

1. Verfahren zur Herstellung von Ketonen der Formel

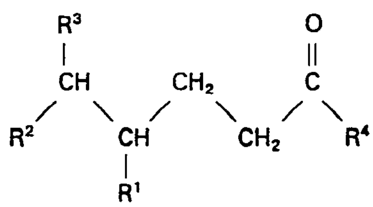

I

in der $R^1$, $R^2$ und $R^3$ Wasserstoffatome, Alkylgruppen mit 1 bis 10 C-Atomen, die durch Alkoxy- oder Arylgruppen substituiert sein können, Arylgruppen, Cycloalkylgruppen oder heterocyclische Reste mit 6 bis 20 C-Atomen, die durch Alkylgruppen substituiert sein können, bedeuten, $R^4$ die gleiche Bedeutung hat, jedoch nicht für ein Wasserstoffatom steht, und $R^1$ und $R^2$ auch Reste sein können, die mit den C-Atomen an die sie gebunden sind, einen cycloaliphatischen oder heterocyclischen Ring bilden, dadurch gekennzeichnet, daß man Aldehyde der Formel

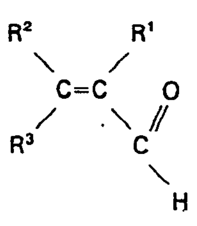

II

in der $R^1$, $R^2$ und $R^3$ die obengenannte Bedeutung haben, mit Ketonen der Formel

$$H_3C—CO—R^4 \qquad III$$

in der $R^4$ die obengenannte Bedeutung hat, bei höherer Temperatur und in einer Stufe in Gegenwart von Wasserstoff und eines Katalysatorsystems, dasu einerseits ein Oxid oder Phosphat der seltenen Erdmetalle, des Magnesiums, Aluminiums, Zinks oder Titans, und andererseits ein Metall der Gruppe VIII des Periodensystems der Elemente enthält, umsetzt.

2. Verfahren nach Anspruch 1, dadurch gekennzeichnet, daß man einen Trägerkatalysator verwendet, der einerseits ein Oxid der seltenen Erdmetalle, des Magnesiums, Aluminiums, Zinks oder Titans und andererseits ein Metall der Gruppe VIII des Periodensystems der Elemente auf einem inerten Träger enthält.

3. Verfahren nach Anspruch 1, dadurch gekennzeichnet, daß man die Umsetzung bei Temperaturen von 100 bis 280°C vornimmt.

**Revendications**

1. Procédé pour la préparation de cétones de formule

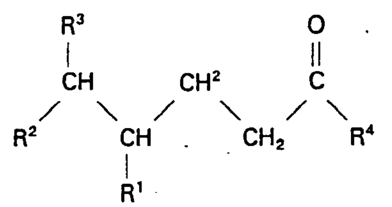

I

dans laquelle $R^1$, $R^2$ et $R^3$ représentent des atomes d'hydrogène, des groupes alkyle à 1—10 atomes de C, qui peuvent être substitués par des groupes alcoxy ou aryle, des groupes aryle, des groupes cycloalkyle ou des radicaux hétérocycliques qui peuvent être substitués par des groupes alkyle, $R^4$ a la même signification, mais n'est pas mis pour un atome d'hydrogène et $R^1$ et $R^2$ peuvent aussi être des radicaux qui forment, avec les atomes de C auxquels ils sont fixés, un noyau cycloaliphatique ou hétérocyclique, caractérisé en ce qu'on fait réagir des aldéhydes de formule

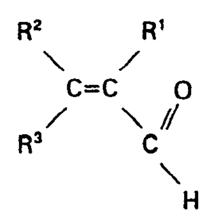

II

dans laquelle $R^1$, $R^2$ et $R^3$ ont les significations données ci-dessus, avec des cétones de formule

$$H_3C—CO—R^4 \qquad III$$

dans laquelle $R^4$ a la signification donnée ci-dessus, à température élevée et en un temps, en

présence d'hydrogène et d'un système catalyseur qui contient d'une part un oxyde ou phosphate des métaux des terres rares, du magnésium, de l'aluminium, du zinc ou du titane et, d'autre part, un métal du groupe VIII du système périodique des éléments.

2. Procédé selon la revendication 2, caractérisé en ce qu'on utilise un catalyseur fixé sur support qui contient, sur un support inerte, d'une part un oxyde des métaux des terres rares, du magnésium, de l'aluminium, du zinc ou du titane et, d'autre part, un métal du groupe VIII du système périodique des éléments.

3. Procédé selon la revendication 1, caractérisé en ce qu'on procédé à la réaction à des températures de 100 à 280°C.

**Claims**

1. A process for the preparation of a ketone of the formula

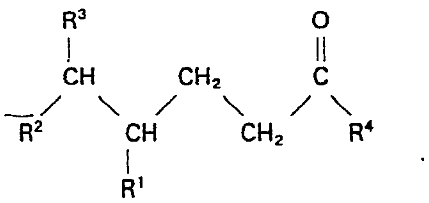

I

where $R^1$, $R^2$ and $R^3$ are each hydrogen, alkyl of 1 to 10 carbon atoms which can be substituted by alkoxy or aryl groups, aryl, cycloalkyl or a heterocyclic radical of 6 to 20 carbon atoms which can be substituted by alkyl groups, $R^4$ has the same meanings but is not hydrogen, and $R^1$ and $R^2$, together with the carbon atoms to which they are bonded, can furthermore form a cycloaliphatic or heterocyclic ring, wherein an aldehyde of the formula

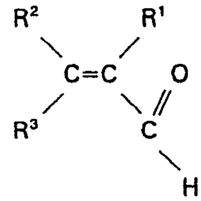

II

where $R^1$, $R^2$ and $R^3$ have the above meanings, is reacted with a ketone of the formula

$$H_3C—CO—R^4 \qquad III$$

where $R^4$ has the above meanings, at elevated temperature and in a single stage, in the presence of hydrogen and of a catalyst system which contains an oxide or phosphate of a rare earth metal, of magnesium, of aluminum, of zinc or of titanium on the one hand, and a metal of group VIII of the periodic table of elements on the other hand.

2. A process as claimed in claim 1, wherein a supported catalyst is used which contains, on an inert carrier, an oxide of a rare earth metal, of magnesium, of aluminum, of zinc or of titanium on the one hand, and a metal of group VIII of the periodic table of elements on the other hand.

3. A process as claimed in claim 1, wherein the reaction is carried out at from 100 to 280°C.